# EUROPÄISCHE PATENTANMELDUNG

(11) **EP 4 020 166 A1**
(43) Veröffentlichungstag der Anmeldung: **29.06.2022**
(21) Anmeldenummer: 20216753.2
(22) Anmeldetag: 22.12.2020
(51) Int. Cl.: G06F 8/65, G06F 8/36, G06F 9/4401, G06F 9/455, G01R 33/54

(54) **VERFAHREN ZUM AUSFÜHREN ZUMINDEST EINER APPLIKATION AUF EINER MAGNETRESONANZVORRICHTUNG**

(71) Anmelder: Siemens Healthcare GmbH, 91052 Erlangen (DE)
(72) Erfinder: Hölscher, Uvo, 91056 Erlangen (DE); Forman, Christoph, 91052 Erlangen (DE); Gall, Peter, 91080 Uttenreuth (DE)

(57) **Zusammenfassung**

Die Erfindung geht aus von einem Verfahren zu einem Ausführen von zumindest einer Applikation auf einer Magnetresonanzvorrichtung, wobei:
- die zumindest eine Applikation auf einer separat ausgebildeten Speichereinheit gespeichert ist,
- die Magnetresonanzvorrichtung eine Schnittstelle umfasst, mittels der eine Kommunikation und/oder ein Datenaustausch mit der zumindest einen Applikation erfolgt,
- mittels eines Informationskanals der Schnittstelle zumindest eine Hardware-Information der Magnetresonanzvorrichtung an die zumindest eine Applikation übermittelt wird,
- Anpassen von zumindest einem Parameter der zumindest einen Applikation an die zumindest eine Hardware-Information der Magnetresonanzvorrichtung, und
- Ausführen der zumindest einen Applikation, wobei mittels eines Steuerkanals der Schnittstelle zumindest eine Steuerinformation von der zumindest einen Applikation an die Magnetresonanzvorrichtung zu einem Ausführen der zumindest einen Applikation übertragen wird.

## Beschreibung

Bei vielen Magnetresonanzvorrichtung ist ein Austausch von Software immer verbunden mit einer Neuinstallation der kompletten Software. Zudem ist die Software speziell auf die Magnetresonanzvorrichtung, insbesondere auf die Hardware-Eigenschaften der Magnetresonanzvorrichtung, zugeschnitten. Derartige Hardware-Eigenschaften sind beispielsweise eine Magnetfeldstärke und/oder eine maximale Gradientenfeldstärke und/oder eine Ausbildung einer Empfangsvorrichtung eines Hochfrequenzsystems usw. Dies führt jedoch dazu, dass ein Austausch der Software sehr aufwendig ist und daher meist nur alle paar Jahre erfolgt. Ein Austausch von einzelnen Softwarekomponenten für eine Magnetresonanzvorrichtung hätte somit auch immer eine komplette Neuinstallation der Software zur Folge, was jedoch sehr aufwendig ist. Dies hat zur Folge, dass die einzelnen Softwarekomponenten gesammelt werden und bei der nächsten Neuinstallation erst berücksichtigt werden. Somit steht eine Anwendung der Softwarekomponenten auch erst nach dieser Neuinstallation der kompletten Software zur Verfügung.

Der vorliegenden Erfindung liegt insbesondere die Aufgabe zugrunde, eine Anwendung von einzelnen Softwarekomponenten unabhängig von einer Installation der Software zu ermöglichen. Die Aufgabe wird durch die Merkmale der unabhängigen Ansprüche gelöst. Vorteilhafte Ausgestaltungen sind in den Unteransprüchen beschrieben.

Die Erfindung geht aus von einem Verfahren zu einem Ausführen von zumindest einer Applikation auf einer Magnetresonanzvorrichtung, wobei:
- die zumindest eine Applikation auf einer separat ausgebildeten Speichereinheit gespeichert ist,
- die Magnetresonanzvorrichtung eine Schnittstelle umfasst, mittels der eine Kommunikation und/oder ein Datenaustausch mit der zumindest einen Applikation erfolgt,
- mittels eines Informationskanals der Schnittstelle zumindest eine Hardware-Information der Magnetresonanzvorrichtung an die zumindest eine Applikation übermittelt wird,
- Anpassen von zumindest einem Parameter der zumindest einen Applikation an die zumindest eine Hardware-Information der Magnetresonanzvorrichtung, und
- Ausführen der zumindest einen Applikation, wobei mittels eines Steuerkanals der Schnittstelle zumindest eine Steuerinformation von der zumindest einen Applikation der Magnetresonanzvorrichtung zu einem Ausführen der zumindest einen Applikation bereitgestellt wird.

Die Magnetresonanzvorrichtung umfasst bevorzugt eine medizinische und/oder diagnostische Magnetresonanzvorrichtung, die zu einem Erfassen von medizinischen und/oder diagnostischen Bilddaten, insbesondere medizinischen und/oder diagnostischen Magnetresonanzbilddaten, eines Patienten ausgelegt und/oder ausgebildet ist. Die Magnetresonanzvorrichtung umfasst hierzu eine Scannereinheit. Die Scannereinheit der Magnetresonanzvorrichtung umfasst bevorzugt eine Detektoreinheit, insbesondere eine Magneteinheit, zur Erfassung der medizinischen und/oder diagnostischen Bilddaten. Bevorzugt umfasst hierbei die Scannereinheit, insbesondere die Magneteinheit, einen Grundmagneten, ein Gradienten-System und eine Hochfrequenzantenneneinheit. Die Hochfrequenzantenneneinheit ist fest innerhalb der Scannereinheit angeordnet und zur Aussendung eine Anregungspulses ausgelegt und/oder ausgebildet. Des Weiteren weist die Magnetresonanzvorrichtung zumindest eine lokalen Hochfrequenzspule auf, die zu einem Empfangen eines Magnetresonanzsignals ausgebildet ist. Hierzu wird die lokale Hochfrequenzspule um den zu untersuchenden Bereich des Patienten angeordnet und/oder angelegt. Bevorzugt sind einzelne lokale Hochfrequenzspulen speziell auf einen Untersuchungsbereich der Patienten ausgelegt, wie beispielsweise eine Kopfhochfrequenzspule oder eine Kniehochfrequenzspule usw.

Der Grundmagnet ist zur Erzeugung eines homogenen Grundmagnetfelds mit einer definierten und/oder bestimmten Magnetfeldstärke, wie beispielsweise mit einer definierten und/oder bestimmten Magnetfeldstärke von 3 T oder 1,5 T usw., ausgebildet. Insbesondere ist der Grundmagnet zur Erzeugung eines starken, konstanten und homogenen Grundmagnetfelds ausgebildet. Das homogene Grundmagnetfeld ist bevorzugt innerhalb eines Patientenaufnahmebereichs der Magnetresonanzvorrichtung angeordnet und/oder vorzufinden. Das Gradienten-System ist zu einer Erzeugung von Magnetfeldgradienten, die für eine Ortskodierung während einer Bildgebung verwendet werden, ausgebildet.

Der Patientenaufnahmebereich ist zu einer Aufnahme des Patienten, insbesondere des zu untersuchenden Bereichs des Patienten, für eine medizinische Magnetresonanzuntersuchung ausgelegt und/oder ausgebildet. Beispielsweise ist hierzu der Patientenaufnahmebereich zylinderförmig ausgebildet und/oder zylinderförmig von der Scannereinheit, insbesondere der Magneteinheit, umgeben. Innerhalb des Patientenaufnahmebereich ist bevorzugt ein Field of View (FOV) und/oder ein Isozentrum der Magnetresonanzvorrichtung angeordnet. Das FOV umfasst bevorzugt einen Erfassungsbereich der Magnetresonanzvorrichtung, innerhalb dessen die Bedingungen für eine Erfassung von medizinischen Bilddaten, insbesondere Magnetresonanzbilddaten, innerhalb des Patientenaufnahmebereichs vorliegen, wie beispielsweise ein homogenes Grundmagnetfeld. Das Isozentrum der Magnetresonanzvorrichtung umfasst bevorzugt den Bereich und/oder Punkt innerhalb der Magnetresonanzvorrichtung, der die optimalen und/oder idealen Bedingungen für die Erfassung von medizinischen Bilddaten, insbesondere Magnetresonanzbilddaten, aufweist. Insbesondere umfasst das Isozentrum den homogensten Magnetfeldbereich innerhalb der Magnetresonanzvorrichtung.

Die Magnetresonanzvorrichtung umfasst zudem eine Systemsteuereinheit, wobei die Systemsteuereinheit zumindest ein Rechenmodul und/oder einen Prozessor umfasst. Die Systemsteuereinheit ist dazu ausgebildet, die einzelnen Komponenten der Magnetresonanzvorrichtung für zumindest eine Magnetresonanzdatenerfassung und/oder eine Magnetresonanzdatenrekonstruktion anzusteuern. Dabei werden von der Systemsteuereinheit entsprechende Steuerbefehle und/oder Steuerdaten für die einzelnen Komponenten der Magnetresonanzvorrichtung in Abhängigkeit von einer mittels des Prozessors und/oder des Rechenmoduls ausgeführten Software generiert. Des Weiteren weist die Magnetresonanzvorrichtung eine Speichereinheit auf, in der eine Software für einen Betrieb der Magnetresonanzvorrichtung gespeichert ist.

So ist insbesondere die Systemsteuereinheit dazu ausgebildet, computerlesbare Instruktionen zur Ansteuerung der einzelnen Komponenten der Magnetresonanzvorrichtung auszuführen. Insbesondere umfasst die Systemsteuereinheit eine Speichereinheit, wobei auf der Speichereinheit computerlesbare Informationen gespeichert sind, wobei die Systemsteuereinheit dazu ausgebildet ist, die computerlesbaren Informationen von der Speichereinheit zu laden und die computerlesbaren Informationen zur Ansteuerung der einzelnen Komponenten der Magnetresonanzvorrichtung auszuführen. Die Komponenten der Systemsteuereinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Selbstverständlich ist es auch denkbar, dass mehrere der genannten Komponenten in Form einer einzelnen Softwarekomponente bzw. softwareunterstützter Hardwarekomponente zusammengefasst realisiert sind.

Eine Applikation richtet sich an eine klare klinische und/oder medizinische Fragestellung, wie beispielsweise "ich möchte Diffusionsbilder der Prostata aufnehmen und daraus eine ADC Karte berechnen". Für verschiedene klinische und/oder medizinische Fragestellungen können somit auch verschiedene Applikationen zur Verfügung stehen, wobei die einzelnen Applikationen unabhängig voneinander sind und sich jede der Applikationen mit bevorzugt einer einzigen klinischen und/oder medizinischen Fragestellung befasst. Die einzelnen Applikationen sind dabei unabhängig voneinander, so dass die einzelnen Applikationen individuell getestet und/oder verteilt und/oder installiert und/oder ausgeführt und/oder desinstalliert werden können. Zudem können die einzelnen Applikationen dabei auch in Applikationspaketen verpackt sein. Ein derartiges Applikationspaket umfasst bevorzugt zwei oder mehr Applikationen, wobei innerhalb des Applikationspakets eine Reihenfolge und/oder ein Workflow der einzelnen Applikationen, insbesondere einer Ausführung der einzelnen Applikationen, nacheinander festgelegt ist. Ein Applikationspaket kann beispielsweise eine Applikation mit einer ersten Magnetresonanzdatenerfassung und eine Applikation mit einer Rekonstruktion der ersten Magnetresonanzdaten sowie eine Applikation mit einer zweiten Magnetresonanzdatenerfassung und eine Applikation mit einer Rekonstruktion der zweiten Magnetresonanzdaten und eine Workflow-Applikation umfassen, wobei die Workflow-Applikation eine Ausführung der einzelnen Applikationen, insbesondere eine Reihenfolge der einzelnen Applikationen steuert. Bevorzugt werden zwischen den einzelnen Applikationen eine Applikationspakets Informationen und/oder Daten ausgetauscht.

Unter der zumindest einen Applikation soll im Sinne der Erfindung insbesondere eine einzige Applikation oder auch mehrere Applikationen oder auch ein Applikationspaket mit mehreren Applikationen verstanden werden.

Die zumindest eine Applikation ist bevorzugt in einer separat ausgebildeten Speichereinheit gespeichert und/oder hinterlegt. Insbesondere ist die Speichereinheit separat zur Speichereinheit der Magnetresonanzvorrichtung, in der eine Software der Magnetresonanzvorrichtung gespeichert ist, ausgebildet. Dabei können die Speichereinheit, in der die zumindest eine Applikation gespeichert ist, und die Speichereinheit für die Software der Magnetresonanzvorrichtung beispielswiese auf einer gleichen Festplatte angeordnet sein, wobei die beiden Bereiche und/oder Speichereinheiten unabhängig und/oder getrennt voneinander ausgebildet sind. Ein Datenaustausch und/oder ein Informationsaustausch zwischen Speichereinheit, in der die zumindest eine Applikation gespeichert ist, und der Systemsteuereinheit der Magnetresonanzvorrichtung ist nur mittels der Schnittstelle möglich. Dabei kann die zumindest eine Applikation in einer als Cloud und/oder als Netzwerk und/oder als Server ausgebildeten Speichereinheit gespeichert und/oder hinterlegt sein. Zudem kann die zumindest eine Applikation auch in einer als Container ausgebildeten Speichereinheit für eine Kommunikation mit der Magnetresonanzvorrichtung zur Verfügung stehen. Unterschiedliche Applikationen können auch auf unterschiedlichen Speicherorten und/oder Speichermedien gespeichert und/oder hinterlegt sein, wobei jeder der unterschiedlichen Applikationen mittels der Schnittstelle mit der Magnetresonanzvorrichtung hinsichtlich eines Informationsaustauschs und/oder eines Datenaustauschs verbunden werden kann. Die einzelnen Applikationen können auch Netzwerk-basiert installiert und/oder gespeichert sein, so dass beispielsweise die einzelnen Applikationen mittels eines Applikations-Webstores verteilt werden können. Die einzelnen Applikationen und/oder Applikationspakete können hierbei technische Pakete umfassen, wie beispielsweise eine virtuelle Maschine und/oder ein Docker Container. Des Weiteren können die einzelnen Applikationen und/oder Applikationspakete auch ein logisches Paket umfassen. Insbesondere verfügt die Schnittstelle und/oder die Magnetresonanzvorrichtung über eine Art Buchhaltung über die einzelnen Applikationen.

Bevorzugt ist die zumindest eine Applikation allgemein formuliert und/oder ausgebildet, so dass die zumindest eine Applikation an unterschiedliche Magnetresonanzvorrichtung angewendet werden kann. Insbesondere ist hierbei die zumindest eine Applikation unabhängig und/oder unspezifisch hinsichtlich von Hardware-Eigenschaften und/oder Hardware-Informationen von Magnetresonanzvorrichtungen ausgebildet. Eine Spezifizierung und/oder eine Anpassung an die mit der zumindest einen Applikation in Kommunikation befindlichen Magnetresonanzvorrichtung erfolgt erst durch die Kommunikation der zumindest einen Applikation mit der Magnetresonanzvorrichtung. Mittels der Kommunikation zwischen der zumindest einen Applikation und der Magnetresonanzvorrichtung werden die Informationen zu den Hardware-Eigenschaften der Magnetresonanzvorrichtung von der zumindest einen Applikation mittels der Schnittstelle abgerufen und/oder eingeholt. Durch ein Abrufen der Informationen zu den spezifischen Hardware-Eigenschaften können einzelne Hardware-spezifische Parameter der zumindest einen Applikation spezifisch und/oder individuell an die Magnetresonanzvorrichtung angepasst werden.

Die zumindest eine Applikation umfasst bevorzugt eine Software, die zur Ausführung an der Magnetresonanzvorrichtung ausgelegt und/oder ausgebildet ist. Mittels der zumindest eine Applikation kann dabei eine definierte Anwendung der Magnetresonanzvorrichtung gesteuert und/oder ausgeführt werden. Bevorzugt umfasst die zumindest eine Applikation dediziert mindestens den Schritt der Magnetresonanzdatenerfassung und/oder der Magnetresonanzdatenakquisition und den Schritt der Magnetresonanzdatenrekonstruktion. Die Magnetresonanzdatenerfassung und/oder die Magnetresonanzdatenakquisition umfasst bevorzugt die Schritte einer Planung der Magnetresonanzdatenerfassung und einer Steuerung der Magnetresonanzdatenerfassung und ist zur Erfassung und/oder Akquirierung von Rohdaten ausgelegt und/oder ausgebildet. Die Magnetresonanzdatenerfassung der zumindest einen Applikation kann dabei zumindest eine Sequenz und/oder zumindest ein Protokoll für eine Magnetresonanzuntersuchung umfassen.

Eine Sequenz für eine Magnetresonanzuntersuchung umfasst insbesondere eine logische Abfolge von Hochfrequenz-Pulsen (HF-Pulsen), Gradientenpulsen und Aufnahme-Zeiträumen zur Steuerung der Datenaufnahme für eine Magnetresonanzdatenerfassung. Die Sequenz legt dabei die grundsätzlichen Magnetresonanzmechanismen fest, die bei der Magnetresonanzdatenerfassung und/oder einer Magnetresonanzdatenakquisition benutzt werden, wie beispielsweise ein Gradienten-Echo oder Spin-Echo, ein Steady-State der Magnetisierung, Präparationspulse usw. Typische und bekannte Sequenzen sind beispielsweise eine Spin-Echo-Sequenz (SE-Sequenz), eine Turbo-Spin-Echo-Sequenz (TSE-Sequenz), eine Echo Planar Imaging (EPI) usw. Eine Sequenz legt jedoch nicht die vollständige zeitliche Ausprägung aller HF-Pulse, Gradientenpulse und Aufnahme-Zeiträume fest, sondern nur deren Zusammenspiel. Grundsätzlich kann eine Parametrisierung einer Sequenz angepasst werden. Hierbei kann insbesondere ein Timing der Sequenz und/oder eine Auflösung der Sequenz an die klinische und/oder medizinische Fragestellung angepasst werden.

Ein Protokoll umfasst dabei einen Satz an Werten, der benötigt wird, um eine konkrete Ausprägung einer Sequenz zu beschreiben. Typische Werte können hierbei eine Echozeit (TE), eine Repetitionszeit (TR), ein Field of View, eine Matrix-Größe, eine Anzahl an Schichten, eine Fettsättigungsmethode, eine Beschleunigungsmethode, eine lokale Hochfrequenzantenneneinheit usw. umfassen. Häufig sind derartige Werte eines Protokolls auch stark von der vor Ort verfügbaren Hardware und/oder Ausgestaltung der Magnetresonanzvorrichtung abhängig, wie beispielsweise von einer Gradientenstärke der Magnetresonanzvorrichtung und/oder einer Anzahl an Aufnahmekanälen usw. Ein Protokoll beschreibt damit eine ganz bestimmte Art und Weise, ein Bild mit einer ganz bestimmten Ausgestaltung einer Magnetresonanzvorrichtung aufzunehmen. Insbesondere sind dabei ein Kontrast und/oder eine Geometrie und eine Messdauer auf die klinische Fragestellung der anstehenden Magnetresonanzuntersuchung abgestimmt.

Die Magnetresonanzdatenrekonstruktion der zumindest einen Applikation ist bevorzugt dazu ausgebildet und/oder ausgelegt, Magnetresonanzbilddaten und/oder weitere Ergebnisse aus den erfassten Magnetresonanzrohdaten zu rekonstruieren. Hierzu kann die zumindest eine Applikation einen Auswertealgorithmus und/oder einen Rekonstruktionsalgorithmus umfassen, der aus erfassten Magnetresonanzrohdaten Magnetresonanzbilddaten und/oder weitere Ergebnisse ermittelt und/oder bestimmt. Bevorzugt sind die Magnetresonanzrohdaten mittels der Magnetresonanzdatenerfassung der zumindest einen Applikation erfasst. Zudem kann die zumindest eine Applikation auch weitere Anwendungen umfassen, wie beispielsweise Post-Processing-Schritte und/oder Workflow-Schritte. Dabei kann eine Post-Processing-Anwendung beispielsweise einen Algorithmus umfassen, der bereits rekonstruierte Magnetresonanzbilddaten weiterverarbeitet. Dabei können aus den bereits rekonstruierten Magnetresonanzbilddaten neue Bilddaten und/oder weitere Ergebnisse aus den bereits rekonstruierten Bilddaten bestimmt und/oder ermittelt werden. Zudem kann ein Workflow einer Applikation und/oder eines Applikationspaketes eine Steuerung, insbesondere eine Ablaufsteuerung und/oder ein Informationsfluss, zwischen einzelnen Sequenzen und/oder Rekonstruktionen umfassen.

Die Schnittstelle umfasst bevorzugt eine universelle Schnittstelle, mittels der Applikationen von unterschiedlichen Speicherorten aus mit einer Magnetresonanzvorrichtung kommunizieren und/oder Daten austauschen können. Die Kommunikation und/oder der Datenaustausch zwischen der zumindest einen Applikation und der Magnetresonanzvorrichtung erfolgt hierbei ausschließlich mittels der Schnittstelle. Bevorzugt ist die Schnittstelle Scanner-unabhängig ausgebildet, so dass eine derartige Schnittstelle für unterschiedlich ausgebildete und/oder ausgestaltete Magnetresonanzvorrichtungen verwendet werden kann. Insbesondere kann dabei die Schnittstelle für alle Scannertypen bei Magnetresonanzvorrichtungen verwendet werden, wobei die Schnittstelle die entsprechenden Hardware-Informationen für die Applikationen zur Verfügung stellt. Insbesondere weist die Schnittstelle eine an der Magnetresonanzvorrichtung angeordneten Schnittstellenelement und ein korrespondierendes, applikationsseitiges Schnittstellenelement auf.

Die Kommunikation und/oder der Datenaustausch zwischen der zumindest einen Applikation und der Magnetresonanzvorrichtung kann dabei sowohl in Richtung von der Magnetresonanzvorrichtung an die zumindest eine Applikation als auch in Richtung von der zumindest einen Applikation an die Magnetresonanzvorrichtung erfolgen. Vorzugsweise weist hierzu die Schnittstelle mehrere Kanäle zu einem Informationsaustausch und/oder Datenaustausch zwischen der zumindest einen Applikation und der Magnetresonanzvorrichtung auf. Die einzelnen Kanäle können dabei Informationskanäle, mittels denen Informationen ausgetauscht werden können, und/oder Steuerkanals, mittels denen Steuerinformationen, beispielsweise Steuerbefehle, ausgetauscht werden können, usw. umfassen. Diese Steuerinformationen können auch erst in der Systemsteuereinheit der Magnetresonanzvorrichtung zu Steuerbefehlen verarbeitet werden. Insbesondere stellt die Schnittstelle eine Abstraktion der Magnetresonanzvorrichtung dar, die alle relevanten Informationen, insbesondere Hardware-Informationen, für die zumindest eine Applikation zur Verfügung stellt.

Die zumindest eine Hardware-Information der Magnetresonanzvorrichtung umfasst bevorzugt Informationen zu spezifischen Hardware-Eigenschaften der Magnetresonanzvorrichtung, wie beispielsweise eine Hardware-Eigenschaft eines Grundmagneten, insbesondere eine Magnetfeldstärke des Grundmagneten, der Magnetresonanzvorrichtung und/oder eine Hardware-Eigenschaft eines Gradienten-Systems, insbesondere eine zur Verfügung stehende Gradientenstärke des Gradienten-Systems, der Magnetresonanzvorrichtung und/oder eine Auswahl an lokalen Hochfrequenzspulen usw. Insbesondere umfasst die zumindest eine Hardware-Information eine minimale Hardware-Eigenschaft, die zur Anpassung der zumindest einen Applikation für eine Ausführung der zumindest einen Applikation erforderlich ist. Somit kann die zumindest eine Hardware-Information eine "Low-Level-Systembeschreibung" der Magnetresonanzvorrichtung umfassen, die alle für die Ausführung der zumindest einen Applikation relevanten Hardware-Informationen enthält. Durch Anpassen von zumindest einem Parameter der zumindest einen Applikation wird die zumindest eine Applikation individuell an die Magnetresonanzvorrichtung, insbesondere an eine zur Verfügung stehende Hardware und/oder aktuelle Hardware-Limitierungen der Magnetresonanzvorrichtung, angepasst.

Für ein Ausführen der zumindest einen Applikation auf der Magnetresonanzvorrichtung werden mittels der Schnittstelle, insbesondere mittels eines Steuerkanals der Schnittstelle, zumindest eine Steuerinformation, bevorzugt mehrere Steuerinformationen für die Magnetresonanzvorrichtung, insbesondere der Systemsteuereinheit der Magnetresonanzvorrichtung, bereitgestellt. Eine Steuerung und/oder eine Kontrolle der Ausführung der zumindest einen Applikation auf der Magnetresonanzvorrichtung erfolgt durch die Applikation, während die Magnetresonanzvorrichtung nur die ausführende Hardware zum Ausführen der zumindest einen Applikation zur Verfügung stellt. Die zumindest eine Steuerinformation kann dabei eine Steuerinformation für die Ansteuerung des Gradienten-Systems, beispielsweise zur Ausspielung eines Gradientenpulses, und/oder eine Steuerinformation für die Hochfrequenzeinheit, beispielsweise zur Ausspielung und/oder Aussendung eines Anregungspulses, und/oder eine Steuerinformation zur Erfassung von Magnetresonanzsignalen usw. umfassen.

Durch die Erfindung kann eine einfache Anwendung, insbesondere eine einfache Installation und/oder Ausführung, von einzelnen Softwarekomponenten und/oder Applikationen ermöglicht werden. Dabei ist die Installation und/oder Ausführung unabhängig von einem Installationsstatus der bereits vorhandenen Software der Magnetresonanzvorrichtung.

Insbesondere können die einzelnen Softwarekomponenten und/oder Applikationen individuell für eine gezielte klinische und/oder medizinische Fragestellung abgerufen werden. Aufgrund der Unabhängigkeit der einzelnen Applikationen und/oder einzelner Applikationspakte kann beispielsweise ein Betreiber einer Magnetresonanzvorrichtung auch nur einzelne Applikationen und/oder einzelne Applikationspakete kaufen, ohne dabei die komplette Software neu zu installieren. Zudem können aufgrund der Unabhängigkeit der einzelnen Applikationen und/oder der einzelnen Applikationspakete auch mehrere Versionen einer Applikation für die Magnetresonanzvorrichtung vorhanden sein, wobei hierzu bevorzugt ein Auswahlmechanismus zur Verfügung steht.

Zudem ist durch die Ausgestaltung der Applikationen auch eine Anwendung bei unterschiedlichen Magnetresonanzvorrichtungen möglich, so dass zusätzliche Entwicklungskosten vorteilhaft eingespart werden können. Des Weiteren können hierdurch auch Applikationen von weiteren Anbietern einfach und schnell auf der Magnetresonanzvorrichtung zur Ausführung gebracht werden.

Ein weiterer Vorteil der Erfindung ist, dass bei einer neu entwickelten Hardware-Funktionalität die Schnittstelle mit einer neuen Funktion versehen werden kann und die "alte Version" der Schnittstelle weiterhin zur Verfügung stehen kann, so dass kein kompletter Austausch der Applikationen erforderlich ist.

In einer vorteilhaften Weiterbildung des erfindungsgemäßen Verfahrens kann es vorgesehen sein, dass die Schnittstelle dazu ausgebildet ist, die für die zumindest eine Applikation erforderlichen Informationen und/oder Daten bereitzustellen und die Information und/oder Daten, die zu Ausführung der zumindest einen Applikation erforderlich sind, der Magnetresonanzvorrichtung bereitzustellen. Die Schnittstelle ist vorzugsweise Scanner-unabhängig ausgebildet, so dass die Schnittstelle bei Magnetresonanzvorrichtung mit unterschiedlichen Ausführungen, insbesondere Hardware-Ausführungen, wie beispielsweise eine Magnetfeldstärke und/oder eine maximale Gradientenstärke und/oder eine Ausführung eines Hochfrequenzsystems usw., angewendet werden kann. Zudem ist die Schnittstelle auch Applikations-unabhängig, so dass die Schnittstelle auch für Applikationen mit unterschiedlichen Ausführungen und/oder Ausgestaltungen verwendet werden kann. Insbesondere ist ein Informationsaustausch zwischen der Magnetresonanzvorrichtung und der zumindest einen Applikation nur mittels der Schnittstelle möglich.

Die Schnittstelle stellt insbesondere eine Abstraktion der Magnetresonanzvorrichtung dar, die alle relevanten Informationen, insbesondere Hardware-Informationen, für die zumindest eine Applikation zur Verfügung stellt. Die Schnittstelle umfasst hierbei eine Art Portal und/oder Plattform für eine Kommunikation, insbesondere einen Informationsaustauch und/oder einen Datenaustausch, zwischen der zumindest einen Applikation und der Magnetresonanzvorrichtung, insbesondere der Systemsteuereinheit der Magnetresonanzvorrichtung.

Die Schnittstelle ist hierbei dazu ausgebildet, der zumindest einen Applikation alle relevanten Informationen, insbesondere eine Hardware-Ausführung der Magnetresonanzvorrichtung, bereitzustellen. Hierzu kann die Schnittstelle eine Speichereinheit aufweisen, in der die relevanten Informationen der Magnetresonanzvorrichtung gespeichert und/oder hinterlegt sind. Beispielsweise sind die relevanten Informationen in einer Tabelle und/oder einer Datenbank hinterlegt. Dabei kann es auch sein, dass die Tabelle und/oder Datenbank dynamisch angepasst wird, wie dies beispielsweise bei sich ändernden Hardware-Informationen der Fall sein kann. So kann insbesondere eine Feldhomogenität innerhalb des Patientenaufnahmebereichs durch Einbringen des Patienten in den Patientenaufnahmebereich eine Änderung erfahren. Zudem kann die Feldinhomogenität unterschiedlich sein bei unterschiedlichen Patienten innerhalb des Patientenaufnahmebereichs der Magnetresonanzvorrichtung. Die Schnittstelle ist bevorzugt dazu ausgebildet, dass alle relevanten und/oder erforderlichen Informationen und/oder Daten, die für ein Ausführen der zumindest einen Applikation auf der Magnetresonanzvorrichtung erforderliche sind, der zumindest einen Applikation bereitgestellt werden.

Des Weiteren werden mittels der Schnittstelle alle für ein Ausführen der zumindest einen Applikation auf der Magnetresonanzvorrichtung erforderlichen Informationen und/oder Daten mittels der Schnittstelle der Magnetresonanzvorrichtung, insbesondere der Systemsteuereinheit der Magnetresonanzvorrichtung, bereitgestellt. Insbesondere können derart der Systemsteuereinheit Steuerinformationen für ein Ansteuern des Gradienten-Systems und/oder des Hochfrequenzsystems bereitgestellt werden.

Insbesondere ist die Schnittstelle über die Zeit konstant ausgebildet, so dass eine Kommunikation zwischen der zumindest einen Applikation und einer Magnetresonanzvorrichtung unabhängig von einem Alter der zumindest einen Applikation und/oder unabhängig von einem Alter der Magnetresonanzvorrichtung erfolgen kann. Des Weiteren kann es sein, dass für eine neue Funktionalität der Magnetresonanzvorrichtung eine neue Schnittstelle, die um diese Funktionalität erweitert ist, bereitgestellt wird. Jedoch können auch Applikationen, die diese neue Funktionalität nicht bedienen, über diese neue Schnittstelle mit der Magnetresonanzvorrichtung kommunizieren.

Für die Kommunikation weist die Schnittstelle bevorzugt mehrere Kanäle auf, wie beispielsweise zumindest einen Informationskanal und/oder zumindest einen Steuerkanal und/oder weitere, dem Fachmann als sinnvoll erscheinende Kanäle zu einem Datenaustausch und/oder Informationsaustausch aufweisen.

In einer vorteilhaften Weiterbildung des erfindungsgemäßen Verfahrens kann es vorgesehen sein, dass die zumindest eine Hardware-Information der Magnetresonanzvorrichtung eine Magnetinformation eines Grundmagneten und/oder eine Gradienteninformation eines Gradienten-Systems und/oder eine Sendeinformation eines Hochfrequenzsystem und/oder eine Empfangsinformation des Hochfrequenzsystems und/oder eine Information einer Rekonstruktionseinheit umfasst. Bevorzugt umfasst die zumindest eine Hardware-Information eine minimale Hardware-Eigenschaft, insbesondere eine "Low-Level-Systembeschreibung" der Magnetresonanzvorrichtung, die zur Anpassung der zumindest einen Applikation für eine Ausführung der zumindest einen Applikation erforderlich ist.

Die Magnetinformation des Grundmagneten kann beispielsweise eine Magnetfeldstärke umfassen. Die Gradienteninformation des Gradienten-Systems kann beispielsweise eine maximal zur Verfügung stehende Gradientenfeldstärke umfassen. Dabei kann die maximal zur Verfügung stehende Gradientenfeldstärke durch die Hardware, also durch die Gradientenspulen des Gradienten-Systems, limitiert sein oder auch durch Beschränkungen und/oder Lizenzen, die eine maximale Gradientenstärke vorgeben, limitiert sein.

Das Hochfrequenzsystem umfasst bevorzugt die Hochfrequenzantenneneinheit und auch die lokal an dem zu untersuchenden Bereich des Patienten angeordneten lokalen Hochfrequenzspulen. Die Sendeinformation des Hochfrequenzsystems kann beispielsweise eine Art und/oder eine Leistung der Hochfrequenzantenneneinheit umfassen. Die Empfangsinformation des Hochfrequenzsystems kann beispielsweise eine Anzahl der mit der Magnetresonanzvorrichtung verbundenen lokalen Hochfrequenzspulen und/oder eine Art der mit der Magnetresonanzvorrichtung verbundenen lokalen Hochfrequenzspulen und/oder eine Spulenauswahl der mit der Magnetresonanzvorrichtung verbundenen lokalen Hochfrequenzspulen und/oder eine Information einer Empfangselektronik usw. umfassen.

Die Information der Rekonstruktionseinheit kann beispielsweise Systeminformationen einer Rekonstruktionsrecheneinheit umfassen.

Derart kann vorteilhaft die zumindest eine Applikation individuell an die vor Ort zur Verfügung stehende Hardware der Magnetresonanzvorrichtung angepasst werden. Insbesondere kann eine universell zur Verfügung stehende Applikation schnell und einfach an eine individuelle Ausbildung einer Magnetresonanzvorrichtung angepasst werden und damit auch eine Anwendungsmöglichkeit für eine Betreiber und/oder einen Benutzer der Magnetresonanzvorrichtung vorteilhaft vergrößert werden.

In einer vorteilhaften Weiterbildung des erfindungsgemäßen Verfahrens kann es vorgesehen sein, dass mittels zumindest eines Informationskanals der Schnittstelle eine von der zumindest einen Applikation bereitgestellte Information zur Darstellung an eine Benutzerschnittstelle der Magnetresonanzvorrichtung bereitgestellt wird. Die bereitgestellte Information kann von der Schnittstelle direkt an die Benutzerschnittstelle übertragen werden oder über die Systemsteuereinheit an die Benutzerschnittstelle weitergeleitet werden.

Die bereitgestellte Information kann beispielsweise eine Information über eine anstehende Datenerfassung und/oder einen aktuellen Status einer Ausführung der zumindest einen Applikation umfassen. Zudem kann die zumindest eine Information auch eine oder mehrere Eingabeaufforderungen an den Benutzer, insbesondere an eine medizinisches Bedienpersonal, umfassen. Beispielsweise können hierbei einstellbare Parameter vom Benutzer an die klinische Fragestellung angepasst werden und/oder es kann eine Starteingabe zum Starten der Applikation eingegeben werden usw.

Die Benutzerschnittstelle der Magnetresonanzvorrichtung umfasst bevorzugt eine Darstellungseinheit und/oder eine Anzeigeeinheit, die beispielsweise einen Monitor und/oder ein Display aufweist. Zudem umfasst die Benutzerschnittstelle auch eine Eingabeeinheit, mittels der eine Eingabe, insbesondere eine Benutzereingabe, erfolgen kann. Die Eingabeeinheit kann dabei eine Tastatur und/oder eine Computermaus umfassen. Zudem kann es auch sein, dass die Darstellungseinheit und die Eingabeeinheit der Benutzerschnittstelle zumindest teilweise einstückig und/oder einteilig miteinander ausgebildet sind, wie beispielsweise mittels eines Touch-Displays und/oder eines berührungsempfindlichen Sensorbildschirms.

Durch diese Ausgestaltung der Erfindung kann eine vorteilhafte Informationsübermittlung an den Benutzer erfolgen. Insbesondere kann derart der Benutzer beispielsweise über einzelne Abschnitte bei einem Ausführen der zumindest einen Applikation informiert werden.

In einer vorteilhaften Weiterbildung des erfindungsgemäßen Verfahrens kann es vorgesehen sein, dass mittels des zumindest einen Informationskanals der Schnittstelle eine Information einer Benutzerinteraktion bezüglich der von der zumindest einen Applikation bereitgestellten Information der zumindest einen Applikation bereitgestellt wird. Dabei kann es vorgesehen sein, dass die von der zumindest einen Applikation bereitgestellte Information eine definierte Benutzerinteraktion mit der Benutzerschnittstelle vorsieht und/oder erlaubt. Die Benutzerinteraktion umfasst bevorzugt eine Eingabe des Benutzers. Die Information einer Benutzerinteraktion umfasst bevorzugt einen Wert und/oder ein Ergebnis der Benutzerinteraktion und/oder der Eingabe des Benutzers an der Benutzerschnittstelle. Dabei kann die Benutzerinteraktion aufgrund einer von der bereitgestellten Information umfassten Eingabeaufforderung getätigten Eingabe an der Benutzerschnittstelle umfassen. Beispielsweise kann hierbei ein Benutzer dem System mitteilen, dass alle Vorbereitungen für die anstehende Ausführung der zumindest einen Applikation abgeschlossen sind und die zumindest eine Applikation nun ausgeführt werden kann. Zudem kann derart auch der Benutzer gezielt zur Eingabe einer Benutzerinterkation mit der zumindest einen Applikation mittels der Benutzerschnittstelle aufgefordert werden.

In einer vorteilhaften Weiterbildung des erfindungsgemäßen Verfahrens kann es vorgesehen sein, dass die von der zumindest einen Applikation bereitgestellte Information eine grafische Oberfläche einer Anzeige umfasst, wobei die grafische Oberfläche mittels der Benutzerschnittstelle der Magnetresonanzvorrichtung darstellbar ist. Dabei stellt die Magnetresonanzvorrichtung die ausführende Hardware, beispielsweise einen Monitor und/oder ein Display der Benutzerschnittstelle der Magnetresonanzvorrichtung, zur Verfügung. Eine Kontrolle und/oder Steuerung der Darstellung, insbesondere der Darstellung der grafischen Oberfläche, mittels der zumindest einen Applikation erfolgt. Insbesondere können derart alle für die Ausführung der zumindest einen Applikation erforderlichen Benutzereingaben besonders einfach und direkt vom Benutzer angefordert werden. Zudem kann derart auch der Benutzer direkt und schnell über die Ausführung der zumindest einen Applikation informiert werden.

In einer vorteilhaften Weiterbildung des erfindungsgemäßen Verfahrens kann es vorgesehen sein, dass die zumindest eine Applikation Scanner-unabhängig aufgebaut ist. Unter einer Scanner-unabhängigen Applikation soll hierbei insbesondere verstanden werden, dass die Applikation unabhängig von technischen Eigenschaften einer Scannereinheit einer Magnetresonanzvorrichtung ausgebildet ist. Eine Spezifizierung und/oder eine Anpassung an die mit der zumindest einen Applikation in Kommunikation befindlichen Magnetresonanzvorrichtung erfolgt erst durch Bereitstellung der zumindest einen Hardware-Information für die zumindest einen Applikation. Hierdurch kann die zumindest eine Applikation für eine Anwendung auf unterschiedlichen Magnetresonanzvorrichtungen und/oder Scannern von Magnetresonanzvorrichtungen bereitgestellt werden und damit ein Scanner-unabhängige Anwendung der zumindest einen Applikation ermöglicht werden. Des Weiteren kann die zumindest eine Applikation besonders schnell und einfach stets an aktuelle Hardware-Limitierungen der Magnetresonanzvorrichtung angepasst werden.

In einer vorteilhaften Weiterbildung des erfindungsgemäßen Verfahrens kann es vorgesehen sein, dass die zumindest eine Applikation für ein Ausführen der zumindest einen Applikation eine Steuerinformation für eine Ansteuerung zumindest einer Hardware-Komponente der Magnetresonanzvorrichtung mittels des Steuerkanals der Schnittstelle bereitstellt. Die zumindest eine Hardware-Komponente der Magnetresonanzvorrichtung umfasst beispielsweise das Gradienten-System und/oder einen Hochfrequenz-Empfangsverstärker und/oder einen Patiententischs der Magnetresonanzvorrichtung. Die Steuerinformationen können hierbei bereits Steuerbefehle umfassen, die direkt von der Systemsteuereinheit der Magnetresonanzvorrichtung zur Ansteuerung der zumindest einen Hardware-Komponenten ausgeführt werden können. Zudem können die Steuerinformationen auch erst innerhalb der Systemsteuereinheit in einen Steuerbefehl zur Ansteuerung der zumindest einen Hardware-Komponente umgesetzt werden. Insbesondere werden von der zumindest einen Applikation bevorzugt Steuerinformationen für alle Hardware-Komponenten, die zur Ausführung der zumindest einen Applikation erforderlich sind, wie beispielsweise zur Erfassung von Magnetresonanzdaten, mittels des Steuerkanals der Schnittstelle bereitgestellt. Hierdurch können die einzelnen Hardware-Komponenten der Scannereinheit der Magnetresonanzvorrichtung optimal während einer Ausführung der zumindest einen Applikation angesteuert werden.

In einer vorteilhaften Weiterbildung des erfindungsgemäßen Verfahrens kann es vorgesehen sein, dass vor einem Ausführen der zumindest einen Applikation und/oder während eines Ausführens der zumindest einen Applikation mittels eines Informationskanals der Schnittstelle dynamische Steuerinformationen der Magnetresonanzvorrichtung der zumindest eine Applikation bereitgestellt werden. Dabei sollen unter dynamischen Steuerinformationen insbesondere Informationen verstanden werden, die dynamisch vor und/oder während der Ausführung der zumindest einen Applikation erfasst werden und die zu einer Steuerung einer Ausführung der zumindest einen Applikation von der zumindest einen Applikation berücksichtigt werden. Beispielsweise können die dynamischen Steuerinformationen EKG-Daten eines Patienten, die von dem Patienten während der Ausführung der zumindest einen Applikation erfasst wird, umfassen. Derartige EKG-Daten können insbesondere als Triggersignal für eine Datenerfassung während eines Ausführens der zumindest einen Applikation dienen. Des Weiteren können die dynamischen Steuerinformationen auch ein Atemsignal des Patienten und/oder eine Bewegungsinformation des Patienten, die während der Ausführung der zumindest einen Applikation erfasst wird, und/oder weitere, dem Fachmann als sinnvoll erscheinende, dynamisch erfassbare Informationen umfassen. Derart kann die Ausführung der zumindest einen Applikation vorteilhaft an einen Patientenzustand und/oder an sich dynamisch verändernde Zustände und/oder Situationen während der Ausführung der zumindest einen Applikation angepasst werden.

In einer vorteilhaften Weiterbildung des erfindungsgemäßen Verfahrens kann es vorgesehen sein, dass mittels eines Informationskanals der Schnittstelle Rohdaten einer Magnetresonanzmessung zur Rekonstruktion der zumindest einen Applikation bereitgestellt werden. Bevorzugt umfassen die Rohdaten mittels der Magnetresonanzvorrichtung erfasste Magnetresonanzrohdaten. Zudem können die Rohdaten auch Rohdaten von Justagemessungen, wie z.B. Messungen zu Spulensensitivitäten von lokalen Hochfrequenzspulen, umfassen. Vorzugsweise wurden die Rohdaten zuvor durch Anwenden der zumindest einen Applikation auf der Magnetresonanzvorrichtung erfasst. Die Rekonstruktion der zumindest einen Applikation ist bevorzugt auf die klinische und/oder medizinische Fragestellung abgestimmt. Insbesondere ist hierbei ein Auswertealgorithmus und/oder einen Rekonstruktionsalgorithmus der zumindest einen Applikation auf die klinische und/oder medizinische Fragestellung abgestimmt, so dass durch die Rekonstruktion der Rohdaten mittels der zumindest einen Applikation eine besonders effiziente und schnelle Rekonstruktion der Rohdaten bezüglich der klinischen und/oder medizinischen Fragestellung erfolgen kann.

In einer vorteilhaften Weiterbildung des erfindungsgemäßen Verfahrens kann es vorgesehen sein, dass mittels des Informationskanals der Schnittstelle rekonstruierte Magnetresonanzbilddaten von der zumindest einen Applikation bereitgestellt werden. Vorzugsweise erfolgt das Bereitstellen der rekonstruierten Magnetresonanzbilddaten unmittelbar nach der Rekonstruktion der Rohdaten mittels der zumindest einen Applikation. Derart kann ein Benutzer besonders schnell Magnetresonanzbilddaten für eine weitere Verwendung und/oder Benutzung erhalten. Insbesondere kann derart ein Benutzer Magnetresonanzbilddaten erhalten, wobei die Rekonstruktion der Magnetresonanzdaten speziell auf die klinische und/oder medizinische Fragestellung abgestimmt ist, und damit eine Befundung und/oder Weiterverarbeitung für den Benutzer erleichtert werden kann.

In einer vorteilhaften Weiterbildung des erfindungsgemäßen Verfahrens kann es vorgesehen sein, dass die Schnittstelle direkt an einer Systemsteuereinheit der Magnetresonanzvorrichtung angeordnet ist und/oder mittels eines Netzwerks mit der Systemsteuereinheit der Magnetresonanzvorrichtung verbunden ist. Das Netzwerk umfasst bevorzugt ein Datennetz zum Austausch von Daten. Dabei kann das Netzwerk von der Magnetresonanzvorrichtung umfasst sein. Zudem ist es auch denkbar, dass es sich bei dem Netzwerk um ein lokales Netzwerk, wie beispielsweise ein Krankenhausnetzwerk, handelt. Derart kann die Schnittstelle besonders einfach an eine vor Ort vorhandene Infrastruktur für eine Magnetresonanzvorrichtung integriert werden. Zudem kann derart eine direkter Datenaustausch der Systemsteuereinheit mit der Schnittstelle erfolgen.

In einer vorteilhaften Weiterbildung des erfindungsgemäßen Verfahrens kann es vorgesehen sein, dass die Schnittstelle eine Lizenzmodul aufweist. Dabei soll unter einem Lizenzmodul insbesondere ein Modul der Schnittstelle verstanden werden, das bevorzugt Lizenzen und/oder Zugriffsrechte, die die Magnetresonanzvorrichtung auf bestimmte Anwendungen und/oder Applikationen hat, verwaltet. Zudem können mittels des Lizenzmoduls auch Zugriffsrechte der einzelnen Applikationen zur Steuerung einzelner Teile der Magnetresonanzvorrichtung verwaltet werden. Mittels des Lizenzmoduls der Schnittstelle kann eine Differenzierung zwischen einzelnen Applikationen erfolgen, indem ein Informationsaustausch und/oder ein Datenaustausch von mittels der Schnittstelle bereitgestellten Informationen und/oder Daten zwischen der Magnetresonanzvorrichtung und einzelnen Applikationen individuell, insbesondere Applikations-abhängig, verwaltet wird. Beispielsweise kann bei einer Übermittlung einer Hardware-Eigenschaft des Gradienten-Systems eine Diffusions-Applikation eine Information mit einer maximalen Gradienten-Stärke des Gradienten-Systems erhalten und eine Perfusions-Applikation nur eine Information mit einer reduzierten Gradienten-Stärke des Gradienten-Systems erhalten. Zudem kann eine Erweiterung einer Lizenz, beispielsweise durch Zukauf von weiteren Rechten, in dem Lizenzmodul entsprechend einfach verwaltet werden.

Des Weiteren kann das Lizenzmodul auch dazu ausgebildet sein, dass eine Nutzung von Applikationen durch eine Magnetresonanzvorrichtung, insbesondere einem Benutzer und/oder Betreiber einer Magnetresonanzvorrichtung, erfasst wird. So kann beispielsweise eine Lizenz mit einer Limitierung an Anwendungen einer Applikation und/oder mit einer Limitierung einer Hardware-Performance einer Hardware-Komponente der Magnetresonanzvorrichtung, wie beispielsweise des Gradienten-Systems der Magnetresonanzvorrichtung, versehen sein. Zudem kann auch eine Limitierung einer Anwendung einer Applikation derart ausgestaltet sein, dass ein Benutzer und/oder ein Betreiber einer Magnetresonanzvorrichtung eine niedrige Stufe der Applikation ohne Zusatzkosten nutzen kann und dagegen eine Nutzung einer höheren Stufe der Applikation einzeln erfasst wird und auch jeweils bezahlt werden muss. Beispielsweise kann hierbei eine niedrige Stufe einer Applikation eine nur geringe Auslastung der Hardware-Komponenten der Magnetresonanzvorrichtung, wie beispielsweise des Gradienten-Systems, umfassen, während die höheren Stufen der Applikation eine entsprechend höhere Auslastung der Hardware-Komponenten der Magnetresonanzvorrichtung und/oder weitere Zusatzleistungen umfassen können.

In einer vorteilhaften Weiterbildung des erfindungsgemäßen Verfahrens kann es vorgesehen sein, dass die Schnittstelle ein Lernmodul aufweist, wobei das Lernmodul ein künstliches neuronales Netzwerk umfasst. Bevorzugt ist das Lernmodul, insbesondere das künstliche neuronale Netzwerk, dazu ausgebildet, eine Anpassung der zumindest einen Applikation an die Magnetresonanzvorrichtung, insbesondere an Hardware-Eigenschaften und/oder an Lizenzlimitierungen der Magnetresonanzvorrichtung, auszuführen.

Bevorzugt basiert die Anpassung der zumindest einen Applikation an die Magnetresonanzvorrichtung, insbesondere an Hardware-Eigenschaften und/oder an Lizenzlimitierungen der Magnetresonanzvorrichtung, auf einem maschinellen Lernverfahren, auch Deep-Learning Verfahren genannt, welches auf dem künstlichen neuronalen Netz basiert. Ein künstliches neuronales Netz (KNN, englisch artificial neural network - ANN) ist insbesondere ein in einem Rechenprogramm nachgebildetes Netz aus künstlichen Neuronen. Das künstliche neuronale Netz basiert dabei typischerweise auf einer Vernetzung von mehreren künstlichen Neuronen. Die künstlichen Neuronen sind dabei typischerweise auf verschiedenen Schichten (layers) angeordnet. Üblicherweise umfasst das künstliche neuronale Netz eine Eingangsschicht und eine Ausgabeschicht (output layer), deren Neuronenausgabe als einzige des künstlichen neuronalen Netzes sichtbar wird. Zwischen der Eingangsschicht und der Ausgabeschicht liegende Schichten werden typischerweise als verdeckte Schichten (hidden layer) bezeichnet. Typischerweise wird zunächst eine Architektur und/oder Topologie eines künstlichen neuronalen Netzes initiiert und dann in einer Trainingsphase für eine spezielle Aufgabe oder für mehrere Aufgaben in einer Trainingsphase trainiert. Das Training des künstlichen neuronalen Netzes umfasst dabei typischerweise eine Veränderung einer Gewichtung einer Verbindung zwischen zwei künstlichen Neuronen des künstlichen neuronalen Netzes. Das Training des künstlichen neuronalen Netzes kann auch eine Entwicklung von neuen Verbindungen zwischen künstlichen Neuronen, ein Löschen von bestehenden Verbindungen zwischen künstlichen Neuronen, ein Anpassen von Schwellwerten der künstlichen Neuronen und/oder ein Hinzufügen oder ein Löschen von künstlichen Neuronen umfassen.

Das künstliche neuronale Netz wurde insbesondere im Vorfeld bereits geeignet für die Anpassung der zumindest einen Applikation an die Magnetresonanzvorrichtung, insbesondere an Hardware-Eigenschaften und/oder an Lizenzlimitierungen der Magnetresonanzvorrichtung, trainiert. Für das Training des künstlichen neuronalen Netzes wurden dabei insbesondere Trainingsdatensätze verwendet, wie beispielsweise Trainingsdaten, die eine minimale Anforderung an die Hardware-Eigenschaften der Magnetresonanzvorrichtung stellen.

Derart kann eine schnelle individuelle und/oder spezifische Anpassungen der zumindest einen Applikation, insbesondere von Protokollen und/oder Sequenzen der zumindest einen Applikation, vorteilhaft erreicht werden. Ein weiterer Vorteil ist, dass die einzelnen Applikationen bei ihrer Entwicklung an keine spezielle Hardware-Eigenschaft von Magnetresonanzvorrichtungen angepasst werden müssen, sondern dass die Applikationen allgemein und/oder universell gehalten werden können. Dies reduziert deutlich einen Entwicklungsaufwand und damit auch Entwicklungskosten für die einzelnen Applikationen.

Des Weiteren geht die Erfindung aus von einer Magnetresonanzvorrichtung mit einer Schnittstelle, wobei die Magnetresonanzvorrichtung mittels der Schnittstelle zu einem Ausführen des Verfahrens zu einem Ausführen von zumindest einer Applikation auf einer Magnetresonanzvorrichtung ausgebildet ist.

Durch die Erfindung kann eine einfache Anwendung, insbesondere eine einfache Installation und/oder Ausführung, von einzelnen Softwarekomponenten und/oder Applikationen ermöglicht werden. Insbesondere können die einzelnen Softwarekomponenten und/oder Applikationen individuell für eine gezielte klinische und/oder medizinische Fragestellung abgerufen werden. Aufgrund der Unabhängigkeit der einzelnen Applikationen und/oder einzelner Applikationspakte kann beispielsweise ein Betreiber einer Magnetresonanzvorrichtung auch nur einzelne Applikationen und/oder einzelne Applikationspakete kaufen, ohne dabei die komplette Software neu zu installieren.

Zudem ist durch die Ausgestaltung der Applikationen auch eine Anwendung bei unterschiedlichen Magnetresonanzvorrichtungen möglich, so dass zusätzliche Entwicklungskosten vorteilhaft eingespart werden können. Des Weiteren können hierdurch auch Applikationen von weiteren Anbietern einfach und schnell auf der Magnetresonanzvorrichtung zur Ausführung gebracht werden.

Die Vorteile der erfindungsgemäßen Magnetresonanzvorrichtung entsprechen im Wesentlichen den Vorteilen des erfindungsgemäßen Verfahrens zu einem Ausführen von zumindest einer Applikation auf einer Magnetresonanzvorrichtung, welche vorab im Detail ausgeführt sind. Hierbei erwähnte Merkmale, Vorteile oder alternative Ausführungsformen können ebenso auf die anderen beanspruchten Gegenstände übertragen werden und umgekehrt.

Des Weiteren geht die Erfindung aus von einem System mit einer Magnetresonanzvorrichtung und zumindest einer Applikation, wobei das System zu einem Ausführen des Verfahrens zu einem Ausführen von zumindest einer Applikation auf einer Magnetresonanzvorrichtung ausgebildet ist.

Die Vorteile des erfindungsgemäßen Systems entsprechen im Wesentlichen den Vorteilen des erfindungsgemäßen Verfahrens zu einem Ausführen von zumindest einer Applikation auf einer Magnetresonanzvorrichtung, welche vorab im Detail ausgeführt sind. Hierbei erwähnte Merkmale, Vorteile oder alternative Ausführungsformen können ebenso auf die anderen beanspruchten Gegenstände übertragen werden und umgekehrt.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: ein System mit einer Magnetresonanzvorrichtung und einer Applikation in einer schematischen Darstellung,
- Fig. 2: ein zu Fig. 1 alternatives System mit einer Magnetresonanzvorrichtung und einer Applikation in einer schematischen Darstellung,
- Fig 3: ein zu den Fig. 1 und 2 alternatives System mit einer Magnetresonanzvorrichtung und einer Applikation in einer schematischen Darstellung,
- Fig. 4: ein Aufbau einer Applikation, und
- Fig. 5: ein erfindungsgemäßes Verfahren zu einem Ausführen von zumindest einer Applikation auf einer Magnetresonanzvorrichtung.

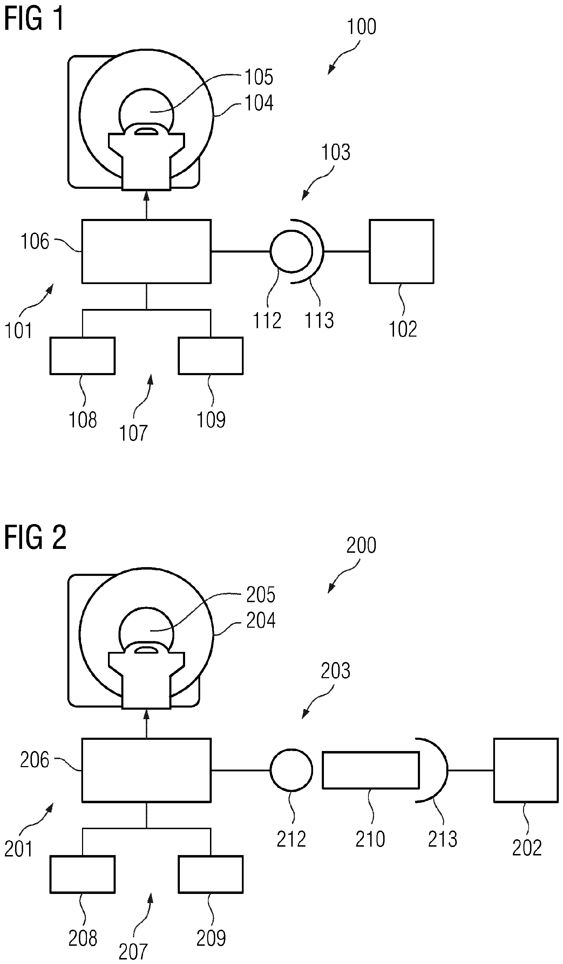

In den Fig. 1 bis 3 ist jeweils ein System 100, 200, 300 mit einer Magnetresonanzvorrichtung 101, 201, 301 und einer Applikation 102, 202, 302 dargestellt, wobei die Magnetresonanzvorrichtung 101, 201, 301 mit der Applikation 102, 202, 302 mittels einer Schnittstelle 103, 203, 303 kommuniziert und/oder Daten und/oder Informationen austauscht.

Die Magnetresonanzvorrichtung 101, 201, 301 umfasst jeweils eine Scannereinheit 104, 204, 304 und einen von der Scannereinheit 104, 204, 304 zylinderförmig umgebenen Patientenaufnahmebereich 105, 205, 305 zur Aufnahme eines Patienten. Die Scannereinheit 104, 204, 304 in den Fig. 1 bis 3 ist nur schematisch dargestellt und umfasst einen supraleitenden Grundmagneten, ein Gradienten-System und ein Hochfrequenzsystem, wie dies aus dem Stand der Technik bekannt ist. Der Grundmagnet zu einem Erzeugen eines starken und insbesondere konstanten Grundmagnetfelds ausgebildet. Das Gradienten-System ist zu einer Erzeugung von Magnetfeldgradienten ausgebildet, die für eine Ortskodierung während einer Bildgebung verwendet werden. Das Hochfrequenzsystem weist eine Hochfrequenzantenne auf zu einer Anregung einer Polarisation ausgebildet, die sich in dem von dem Grundmagneten erzeugten Grundmagnetfeld einstellt.

Zu einer Steuerung des Grundmagneten, des Gradienten-Systems und zur Steuerung der Hochfrequenzantenneneinheit weist die Magnetresonanzvorrichtung 101, 201, 301 jeweils eine Systemsteuereinheit 106, 206, 306 auf. Die Systemsteuereinheit 106, 206, 306 steuert zentral die Magnetresonanzvorrichtung 101, 201, 301.

Des Weiteren umfasst die Magnetresonanzvorrichtung 101, 201, 301 eine Benutzerschnittstelle 107, 207, 307, die mit der Systemsteuereinheit 106, 206, 306 verbunden ist. Steuerinformationen wie beispielsweise Bildgebungsparameter, sowie rekonstruierte Magnetresonanzbilder können auf einer Darstellungseinheit 108, 208, 308, beispielsweise auf zumindest einem Monitor, der Benutzerschnittstelle 107, 207, 307 für ein medizinisches Bedienpersonal angezeigt werden. Weiterhin weist die Benutzerschnittstelle 107, 207, 307 eine Eingabeeinheit 109, 209, 309 auf, mittels der Informationen und/oder Parameter während eines Messvorgangs von dem medizinischen Bedienpersonal eingegeben werden können.

Die Schnittstelle 103, 203, 303 des Systems 100, 200, 300 ist hierbei als universelle Schnittstelle 103, 203, 303 ausgebildet, so dass mittels der Schnittstelle 103, 203, 303 unterschiedliche Applikationen 102, 202, 302 und/oder Softwarepakete mit der Magnetresonanzvorrichtung 101, 201, 301 kommunizieren und/oder Daten austauschen können. Insbesondere ist hierbei die Schnittstelle 103, 203, 303 sowohl Scanner-unabhängig als auch Applikations-unabhängig ausgebildet. Die Schnittstelle 103, 203, 303 umfasst dabei eine Art Plattform oder Portal für eine Kommunikation, insbesondere einen Informationsaustauch und/oder einen Datenaustausch, zwischen der Applikation 102, 202, 302 und der Magnetresonanzvorrichtung 101, 201, 301, insbesondere der Systemsteuereinheit 106, 206, 306 der Magnetresonanzvorrichtung 101, 201, 301. Die Schnittstelle 103, 203, 303 weist hierzu ein erstes Schnittstellenelement 112, 212, 312 das an der Magnetresonanzvorrichtung angeordnet ist und ein zweites, korrespondierendes Schnittstellenelement 113, 213, 313, das applikationsseitig angeordnet ist, auf.

Der Informationsaustausch und/oder der Datenaustausch zwischen der Applikation 102, 202, 302 und der Magnetresonanzvorrichtung 101, 201, 301 kann dabei sowohl in Richtung von der Magnetresonanzvorrichtung 101, 201, 301 an die zumindest eine Applikation 102, 202, 302 als auch in Richtung von der zumindest einen Applikation 102, 202, 302 an die Magnetresonanzvorrichtung 101, 201, 301 mittels der Schnittstelle 103, 203, 303 erfolgen. Insbesondere kann der Informationsaustausch und/oder der Datenaustausch mittels der Schnittstelle 103, 203, 303 bidirektional erfolgen. Vorzugsweise weist hierzu die Schnittstelle 103, 203, 303 mehrere Kanäle zu einem Informationsaustausch und/oder Datenaustausch zwischen der Applikation 102, 202, 302 und der Magnetresonanzvorrichtung 101, 201, 301 auf. Die einzelnen Kanäle können dabei Informationskanäle, mittels denen Informationen ausgetauscht werden können, und/oder Steuerkanäle, mittels denen Steuerinformation, beispielsweise Steuerbefehle, ausgetauscht werden können, usw. umfassen.

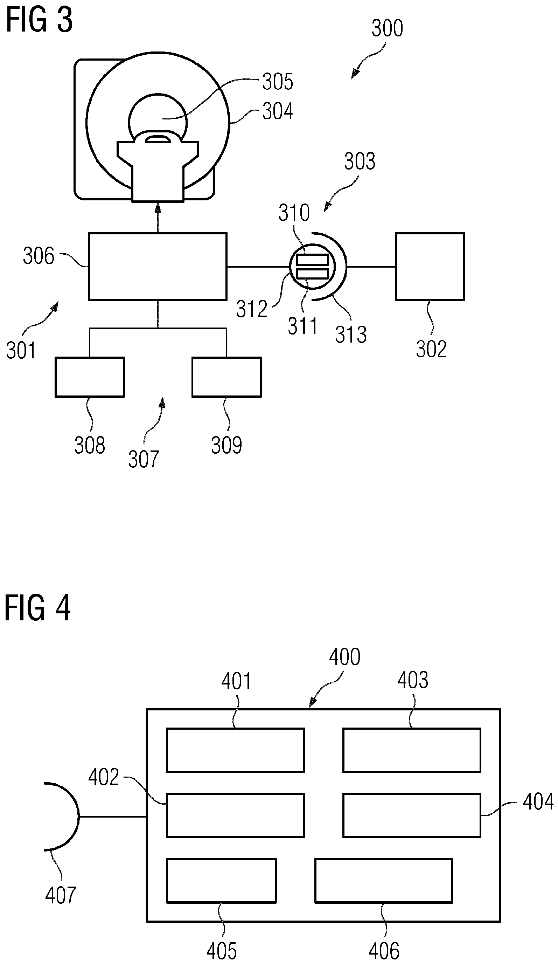

Die in den Fig. 1 bis 3 dargestellten Systeme 100, 200, 300 unterscheiden sich hinsichtlich einer Ausgestaltung der Schnittstelle 103, 203, 303. Hingegen weisen die Magnetresonanzvorrichtungen 101, 201, 301 und die Applikationen 102, 202, 302 einen im Wesentlichen identischen Aufbau und/oder eine im Wesentlichen identische Ausgestaltung auf. Die Schnittstelle 103, 303 in den Fig. 1 und 3 koppeln dabei direkt an die Systemsteuereinheit 106, 306 der Magnetresonanzvorrichtung 101, 301. Die Schnittstelle 203 in Fig. 2 ist mittels eines Netzwerks 210 mit der Magnetresonanzvorrichtung 201, insbesondere der Systemsteuereinheit 206 der Magnetresonanzvorrichtung 201 verbunden. In Fig. 3 weist die Schnittstelle 303 zusätzlich noch ein Lizenzmodul 310 und ein Lernmodul 311 auf. Das Lizenzmodul 310 und das Lernmodul 310 sind dabei von dem Schnittstellenelement 312 der Magnetresonanzvorrichtung 301 umfasst. Eine Funktionsweise dieser beiden Module wird weiter unten noch näher beschrieben.

Die Applikation 102, 202, 302 ist auf einer separat ausgebildeten Speichereinheit gespeichert und/oder hinterlegt. Die zumindest eine Speichereinheit ist hierbei bevorzugt separat zu einer Systemsteuereinheit 106, 206, 306 und separat zu einer Speichereinheit, in der eine Software der Magnetresonanzvorrichtung 101, 201, 301 gespeichert ist, der Magnetresonanzvorrichtung 101, 201, 301 ausgebildet. Die Speichereinheit, in der die Applikation 102, 202, 302 gespeichert und/oder hinterlegt ist, kann dabei auch von einer Cloud und/oder einem Netzwerk und/oder einem Server und/oder einem Container umfasst sein.

Eine Applikation richtet 102, 202, 302 sich an eine klare klinische und/oder medizinische Fragestellung. Für verschiedene klinische und/oder medizinische Fragestellungen können somit auch verschiedene Applikationen 102, 202, 302 zur Verfügung stehen, wobei die einzelnen Applikationen 102, 202, 302 unabhängig voneinander sind und sich jede der Applikationen 102, 202, 302 mit einer eigenen klinischen und/oder medizinischen Fragestellung befasst.

In Fig. 4 ist ein Applikationspaket 400 näher dargestellt. Das Applikationspaket 400 umfasst bevorzugt eine Software, die zur Ausführung an der Magnetresonanzvorrichtung 101, 201, 301 ausgelegt und/oder ausgebildet ist. Das Applikationspaket in Fig. 4 weist mehrere einzelnen Applikationen 401, 402, 403, 404, 405, 406 und ein Schnittstellenelement 407 auf. Mittels der Applikation 401, 402, 403, 404, 405, 406 kann dabei ein definierte Anwendung der Magnetresonanzvorrichtung 101, 201, 301 gesteuert und/oder ausgeführt werden. Das Applikationspaket 400 umfasst zwei Applikationen 401, 402 zur Magnetresonanzdatenerfassung und/oder Magnetresonanzdatenakquisition und zwei Applikationen 403, 404 zur Magnetresonanzdatenrekonstruktion 403, 404. Die beiden Applikationen 401, 402 der Magnetresonanzdatenerfassung umfassen bevorzugt die Schritte einer Planung der Datenakquisition an der Magnetresonanzvorrichtung 101, 201, 301 und einer Steuerung der Datenakquisition an der Magnetresonanzvorrichtung 101, 201, 301 zur Erfassung und/oder Akquirierung von Rohdaten. Die beiden Applikationen 401, 402 der Magnetresonanzdatenerfassung umfassen dabei zumindest eine Sequenz und/oder zumindest ein Protokoll für eine Magnetresonanzuntersuchung.

Die beiden Applikationen 403, 404 der Magnetresonanzdatenrekonstruktion umfassen bevorzugt einen Auswertealgorithmus und/oder einen Rekonstruktionsalgorithmus, der aus den erfassten Magnetresonanzrohdaten Magnetresonanzbilddaten und/oder weitere Ergebnisse ermittelt. Eine weitere Applikation 405 des Applikationspakets 400 umfasst eine Post-Processing-Applikation. Eine derartige Post-Processing-Applikation kann dabei einen Algorithmus, insbesondere einen Post-Processing-Algorithmus, umfassen, der aus Magnetresonanzbilddaten neu Bilddaten und/oder weitere Ergebnisse erzeugt und/oder bestimmt. Das Applikationspaket 400 weist des Weiteren eine weitere Applikation 406 auf, wobei die weitere Applikation 406 eine Workflow-Applikation umfasst. Die Workflow-Applikation ist bevorzugt dazu ausgebildet, einen Ablauf und/oder eine Ausführungsreihenfolge der einzelnen Applikationen 401, 402, 403, 404, 405 des Applikationspakets zu steuern und/oder einen Informationsaustausch zwischen den einzelnen Applikationen 401, 402, 403, 404, 405 zu steuern. Zudem kann das Applikationspaket 400 in einer alternativen Ausführung auch weitere, dem Fachmann als sinnvoll erscheinende Applikationen aufweisen.

Die einzelnen Applikationen 401, 402, 403, 404, 405, 406 sind Scanner-unabhängig ausgebildet und/oder aufgebaut. Insbesondere sind hierbei die einzelnen Applikation 401, 402, 403, 404, 405, 406 unabhängig von individuellen Eigenschaften, wie beispielsweise Hardware-Eigenschaften, und/oder einer technischen Ausgestaltung von Magnetresonanzvorrichtungen 101, 201, 301 ausgebildet und/oder aufgebaut. Erst durch einen Datenaustausch und/oder einen Informationsaustausch mit der Magnetresonanzvorrichtung 101, 201, 301 mittels der Schnittstelle 103, 203, 303 erfolgt eine individuelle und/oder spezifischen Anpassung der einzelnen Applikation 401, 402, 403, 404, 405, 406, insbesondere eine individuelle und/oder spezifischen Anpassung der Sequenzen und/oder Protokolle der einzelnen Applikationen 401, 402, 403, 404, 405, 406.

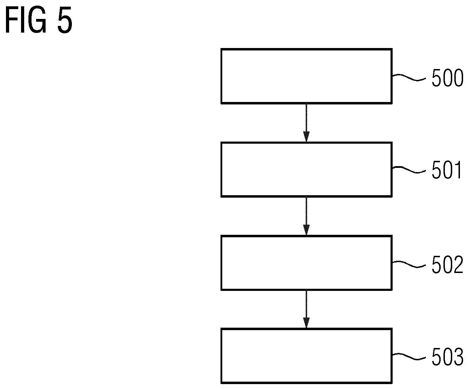

In Fig. 5 ist ein erfindungsgemäßes Verfahren zu einem Ausführen einer Applikation 102, 202, 302 auf einer Magnetresonanzvorrichtung 101, 201, 301 dargestellt. Für eine Magnetresonanzuntersuchung an einem Patienten liegt eine konkrete medizinische und/oder klinische Fragestellung vor. Diese medizinische und/oder klinische Fragestellung soll mittels erfasster Magnetresonanzdaten, die mittels der Magnetresonanzuntersuchung am Patienten, insbesondere am zu untersuchenden Bereich des Patienten, erfasst werden, geklärt werden. Hierzu stehen einem medizinischen Bedienpersonal, beispielsweise einen die Magnetresonanzuntersuchung betreuendem Arzt, ein oder mehrere Untersuchungsabläufe zur Verfügung. Im vorliegenden Fall wählt das medizinische Bedienpersonal in einem ersten Schritt 500 eine Applikation 102, 202, 302 aus, die wie oben beschrieben, auf einer separat ausgebildeten Speichereinheit gespeichert ist. Die Auswahl der Applikation 102, 202, 302 kann dabei mittels der Benutzerschnittstelle 107, 207, 307 der Magnetresonanzvorrichtung 101, 201, 301 erfolgen. Gesteuert wird die Auswahl durch die Systemsteuereinheit 106, 206, 306 der Magnetresonanzvorrichtung 101, 201, 301.

Sobald eine Applikation 102, 202, 302 oder auch mehrere Applikationen 102, 202, 302 oder ein Applikationspaket 400 vom Benutzer ausgewählt wurden, erfolgt in einem weiteren Schritt 501 eine Kommunikation und/oder ein Datenaustausch zwischen der Magnetresonanzvorrichtung 101, 201, 301 und der zumindest einen Applikation 102, 202, 302, insbesondere der einen Applikation 102, 202, 302 oder auch der mehreren Applikationen 102, 202, 302. Dabei wird mittels eines Informationskanals der Schnittstelle 103, 203, 303 zumindest eine Hardware-Information der Magnetresonanzvorrichtung 101, 201, 301 an die zumindest eine Applikation 102, 202, 302 bereitgestellt. Das Bereitstellen der zumindest einen Hardware-Information erfolgt automatisch mittels der zumindest einen Applikation 102, 202, 302 und/oder mittels der Schnittstelle 103, 203, 303. Für das Bereitstellen der zumindest einen Hardware-Information kann die Schnittstelle 103, 203, 303 auch eine Datenbank und eine Tabelle umfassen und/oder auf eine Datenbank und/oder eine Tabelle zugreifen, die in einer Speichereinheit der Schnittstelle 103, 203, 303 und/oder der Systemsteuereinheit 106, 206, 306 gespeichert ist. Hierbei stellt die Schnittstelle 103, 203, 303 eine Abstraktion der Magnetresonanzvorrichtung 101, 201, 301 dar, die alle relevanten Informationen, insbesondere Hardware-Informationen, für die zumindest eine Applikation 103, 203, 303 zur Verfügung stellt.

Die zumindest eine Hardware-Information der Magnetresonanzvorrichtung 101, 201, 301 umfasst dabei eine spezifische und/oder individuelle Hardware-Information der Magnetresonanzvorrichtung 101, 201, 301. Die zumindest eine Hardware-Information der Magnetresonanzvorrichtung 101, 201, 301 kann eine Magnetinformation des Grundmagneten der Magnetresonanzvorrichtung 101, 201, 301 umfassen. Bevorzugt umfasst die Magnetinformation dabei eine Magnetfeldstärke eines mittels des Grundmagneten erzeugten und/oder generierten Grundmagnetfelds. Beispielsweise kann die Magnetfeldstärke 1,5 T oder 3 T usw. umfassen.

Zudem kann die zumindest eine Hardware-Information auch eine Gradienteninformation des Gradienten-Systems umfassen. Die Gradienteninformation des Gradienten-Systems kann beispielsweise eine maximal zur Verfügung stehende Gradientenfeldstärke umfassen. Dabei kann die maximal zur Verfügung stehende Gradientenfeldstärke durch die Hardware, also durch Gradientenspulen des Gradienten-Systems, vorgegeben sein oder auch durch Beschränkungen und/oder Lizenzen, die eine maximal ausspielbare Gradientenfeldstärke vorgeben, limitiert sein.

Des Weiteren kann die zumindest eine Hardware-Information auch eine Sendeinformation des Hochfrequenzsystems und/oder eine Empfangsinformation des Hochfrequenzsystems umfassen. Das Hochfrequenzsystem umfasst bevorzugt die Hochfrequenzantenne und auch die lokal an dem zu untersuchenden Bereich des Patienten angeordneten lokalen Hochfrequenzspulen. Die Sendeinformation des Hochfrequenzsystems kann beispielsweise eine Art und/oder eine Leistung der Hochfrequenzantenne umfassen. Die Empfangsinformation des Hochfrequenzsystems kann beispielsweise eine Anzahl der mit der Magnetresonanzvorrichtung verbundenen lokalen Hochfrequenzspulen und/oder eine Art der mit der Magnetresonanzvorrichtung verbundenen lokalen Hochfrequenzspulen und/oder eine Spulenauswahl der mit der Magnetresonanzvorrichtung verbundenen lokalen Hochfrequenzspulen und/oder eine Information einer Empfangselektronik usw. umfassen.

Weiterhin kann die Hardware-Information der Magnetresonanzvorrichtung 101, 201, 301 auch eine Information einer Rekonstruktionseinheit umfassen. Die Information der Rekonstruktionseinheit kann beispielsweise Systeminformationen einer Rekonstruktionsrecheneinheit umfassen.

Anschließend erfolgt in einem weiteren Schritt 502 eine Anpassen von zumindest einem Parameter der zumindest einen Applikation 102, 202, 302 an die zumindest eine Hardware-Information der Magnetresonanzvorrichtung 101, 201, 301. Dabei erfolgt durch Anpassen des zumindest einen Parameters eine individuelle Anpassung der zumindest einen Applikation 102, 202, 302 an eine vor Ort zur Verfügung stehende Hardware und/oder Magnetresonanzvorrichtung 101, 201, 301 für die Ausführung der zumindest einen Applikation 102, 202, 302 auf der Magnetresonanzvorrichtung 101, 201, 301.

Anschließend erfolgt in einem weiteren Schritt 503 ein Ausführen der zumindest einen Applikation 102, 202, 302, wobei mittels eines Steuerkanals der Schnittstelle 103, 203, 303 zumindest eine Steuerinformation von der zumindest einen Applikation 102, 202, 302 für die Magnetresonanzvorrichtung 101, 201, 301 zu einem Ausführen der zumindest einen Applikation 102, 202, 302 bereitgestellt wird. Ein Ausführen der zumindest einen Applikation 102, 202, 302 umfasst bevorzugt eine Erfassung oder Akquisition von Magnetresonanzdaten und eine Rekonstruktion der erfassten Magnetresonanzdaten. Zudem kann das Ausführen der zumindest einen Applikation 102, 202, 302 auch weitere Post-Processing-Schritte oder Workflow-Schritte usw. umfassen. Die zumindest eine Steuerinformation, die von der zumindest einen Applikation 102, 202, 302 über die Schnittstelle 103, 203, 303 bereitgestellt wird, ist an die Systemsteuereinheit 106, 206, 306 gerichtet, die vor Ort eine Ansteuerung der einzelnen Komponenten der Magnetresonanzvorrichtung 101, 201, 301 gemäß den Vorgaben durch die zumindest eine Applikation 102, 202, 302 durchführt. Beispielsweise wird bei einem Ausführen der zumindest einen Applikation 102, 202, 302 durch die bereitgestellten Steuerinformationen eine direkte Ansteuerung des Gradienten-Systems der Magnetresonanzvorrichtung 101, 201, 301 und/oder des Hochfrequenzsystems und/oder einer Positionierung eines Patiententischs der Magnetresonanzvorrichtung 101, 201, 301 von der Systemsteuereinheit 106, 206, 306 gesteuert. Bevorzugt werden von der zumindest einen Applikation 102, 202, 302 für alle Hardware-Komponenten, die zur Ausführung der zumindest einen Applikation 102, 202, 302 erforderlich sind, Steuerinformation an der Systemsteuereinheit 106, 206, 306 mittels des Steuerkanals der Schnittstelle 103, 203, 303 bereitgestellt.

Während des Ausführens der zumindest einen Applikation 102, 202, 302 kann zudem mittels des Informationskanals der Schnittstelle 103, 203, 303 eine von der zumindest einen Applikation 102, 202, 302 bereitgestellte Information zur Darstellung an der Benutzerschnittstelle 107, 207, 307 der Magnetresonanzvorrichtung 101, 201, 301 an der Magnetresonanzvorrichtung 101, 201, 301 bereitgestellt werden. Hierbei wird insbesondere von der zumindest einen Applikation 102, 202, 302 festgelegt, welche Parameter und/oder welche Informationen auf der Darstellungseinheit 108, 208, 308 der Benutzerschnittstelle 107, 207, 307 für den Benutzer, insbesondere einem medizinischen Bedienpersonal, angezeigt oder dargestellt werden. Dabei können von der zumindest einen Applikation 102, 202, 302 nur Parameter und/oder Informationen ausgewählt werden, die auf einer grafischen Benutzeroberfläche, die von der Benutzerschnittstelle 107, 207, 307 und/oder der Systemsteuereinheit 106, 206, 306 der Magnetresonanzvorrichtung 101, 201, 301 zur Verfügung gestellt wird, mittels der Darstellungseinheit 108, 208, 308 dargestellt werden. Zudem kann es auch sein, dass die von der zumindest einen Applikation 102, 202, 302 bereitgestellte Information auch eine grafischen Oberfläche einer Anzeige umfasst, die von der zumindest einen Applikation 102, 202, 302 zur Anzeige und/oder Darstellung auf der Benutzerschnittstelle 107, 207, 307 der Magnetresonanzvorrichtung 101, 201, 301 bereitgestellt wird.

Des Weiteren wird kann hierbei mittels des zumindest eines Informationskanals der Schnittstelle 103, 203, 303 eine Benutzerinteraktion bezüglich der von der zumindest einen Applikation 102, 202, 302 bereitgestellten Information an die zumindest eine Applikation 102, 202, 302 übermittelt und/oder bereitgestellt werden. Dabei kann es vorgesehen sein, dass die von der zumindest einen Applikation 102, 202, 302 bereitgestellte Information eine definierte Benutzerinteraktion mit der Benutzerschnittstelle erlaubt und/oder erfordert. Beispielsweise kann die von der zumindest einen Applikation bereitgestellte Information auch eine Eingabeaufforderung an den Benutzer umfassen, wobei die Eingabeaufforderung eine Benutzerinteraktion, insbesondere eine Eingabe, an der Benutzerschnittstelle 107, 207, 307 der Magnetresonanzvorrichtung 101, 201, 301 zur Folge hat. Beispielsweise kann hierbei ein Benutzer der zumindest einen Applikation 102, 202, 302 mitteilen, dass alle Vorbereitungen für die anstehende Ausführung der zumindest einen Applikation 102, 202, 302 abgeschlossen sind und die zumindest eine Applikation 102, 202, 302 nun ausgeführt werden kann. Des Weiteren kann eine Benutzerinteraktion auch eine Starteingabe für ein Starten der Magnetresonanzdatenerfassung umfassen. Eine derartige Starteingabe kann beispielsweise abhängige sein von einer Position des Patienten und/oder einer Anordnung von Zubehöreinheiten, die nur der Benutzer vor Ort überprüfen muss. Zudem kann derart auch der Benutzer gezielt zur Eingabe einer Benutzerinterkation mit der zumindest einen Applikation 102, 202, 302 mittels der Benutzerschnittstelle 107, 207, 307 aufgefordert werden.

Zudem können mittels des Informationskanals der Schnittstelle 103, 203, 303 vor einem Ausführen der zumindest einen Applikation 102, 202, 302 und/oder auch während des Ausführens der zumindest einen Applikation 102, 202, 302 dynamische Steuerinformationen von der Magnetresonanzvorrichtung 101, 201, 301 der zumindest eine Applikation 102, 202, 302 bereitgestellt werden. Derartige dynamische Steuerinformationen umfassen dabei Informationen, die bevorzugt fortlaufend erfasst werden und die zur Steuerung einer Ausführung der zumindest einen Applikation 102, 202, 302 von der zumindest einen Applikation 102, 202, 302 berücksichtigt werden. Beispielsweise umfassen die dynamischen Steuerinformationen EKG-Daten des Patienten und/oder ein Atemsignal des Patienten und/oder eine Herzrate des Patienten und/oder weitere, sich fortlaufend ändernde und/oder sich dynamisch ändernde Informationen. Eine derartige dynamische Steuerinformation, wie beispielsweise EKG-Daten des Patienten, kann als Triggersignal für die Erfassung und/oder Akquirierung von Magnetresonanzdaten von der zumindest einen Applikation 102, 202, 302 verwendet werden.

Für eine Datenrekonstruktion, insbesondere eine Magnetresonanzdatenrekonstruktion, werden bei einem Ausführen der zumindest einen Applikation 102, 202, 302 mittels des Informationskanals der Schnittstelle 103, 203, 303 Rohdaten einer Magnetresonanzmessung, insbesondere einer Erfassung und/oder Akquisition von Magnetresonanzdaten, von der Magnetresonanzvorrichtung 101, 201, 301 der zumindest einen Applikation 102, 202, 302 bereitgestellt. Innerhalb der zumindest einen Applikation 102, 202, 302 erfolgt dann bevorzugt die Rekonstruktion der Rohdaten, insbesondere der Magnetresonanzrohdaten. Des Weiteren können anschließend die rekonstruierten Magnetresonanzdaten, insbesondere Magnetresonanzbilddaten, von der zumindest einen Applikation 102, 202, 302 der Magnetresonanzvorrichtung 101, 201, 301 mittels des Informationskanals der Schnittstelle 103, 203, 303 bereitgestellt und/oder übertragen werden.

Weist die Schnittstelle 303 zudem ein Lizenzmodul 310 auf, wie dies in Fig. 3 dargestellt ist, können mittels der Schnittstelle 303, insbesondere mittels des Lizenzmoduls 310 der Schnittstelle 303, Lizenzen und/oder Zugriffsrechte verwaltet werden. Dabei werden mittels des Lizenzmoduls 310 der Schnittstelle 303 sowohl Lizenzen und/oder Zugriffsrechte, die die Magnetresonanzvorrichtung 301 auf bestimmte Anwendungen und/oder Applikationen 302 hat, verwaltet, als auch Zugriffsrechte der einzelnen Applikationen 302 zur Steuerung einzelner Teile der Magnetresonanzvorrichtung 301 verwaltet. Mittels des Lizenzmoduls 310 kann innerhalb der Schnittstelle 303 eine Differenzierung zwischen einzelnen Applikationen 302 erfolgen, indem ein Informationsaustausch und/oder ein Datenaustausch zwischen der Magnetresonanzvorrichtung 301 und einzelnen Applikationen 302 individuell, insbesondere Applikations-abhängig, von dem Lizenzmodul 310 verwaltet wird. Beispielsweise kann bei einer Übermittlung einer Hardware-Eigenschaft des Gradienten-Systems eine Diffusions-Applikation eine Information mit einer maximalen Gradienten-Stärke des Gradienten-Systems erhalten und eine Perfusions-Applikation nur eine Information mit einer reduzierten Gradienten-Stärke des Gradienten-Systems erhalten. Zudem kann eine Erweiterung einer Lizenz, beispielsweise durch Zukauf von weiteren Rechten, in dem Lizenzmodul 310 entsprechend verwaltet werden. Des Weiteren kann das Lizenzmodul 310 auch dazu ausgebildet sein, dass eine Nutzung von Applikationen 302, insbesondere eine Anzahl an Ausführungen einer Applikation 302, erfasst wird.

Weist die Schnittstelle 303 zudem ein Lernmodul 311 auf, wie dies in Fig. 3 dargestellt ist, kann mittels der Schnittstelle 303, insbesondere des Lernmoduls 311 der Schnittstelle 303, eine Anpassung der zumindest einen Applikation 302 an die Magnetresonanzvorrichtung 301, insbesondere an Hardware-Eigenschaften und/oder an Lizenzlimitierungen der Magnetresonanzvorrichtung 301, automatisch und/oder selbsttätig ausgeführt werden. Das Lernmodul umfasst bevorzugt eine trainiertes künstliches neuronales Netzwerk, das hinsichtlich einer Anpassung einer Applikation 302 an die Hardware-Eigenschaften der Magnetresonanzvorrichtung 301 trainiert ist.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zu einem Ausführen von zumindest einer Applikation auf einer Magnetresonanzvorrichtung, wobei:
- die zumindest eine Applikation auf einer separat ausgebildeten Speichereinheit gespeichert ist,
- die Magnetresonanzvorrichtung eine Schnittstelle umfasst, mittels der eine Kommunikation und/oder ein Datenaustausch mit der zumindest einen Applikation erfolgt,
- mittels eines Informationskanals der Schnittstelle zumindest eine Hardware-Information der Magnetresonanzvorrichtung an die zumindest eine Applikation übermittelt wird,
- Anpassen von zumindest einem Parameter der zumindest einen Applikation an die zumindest eine Hardware-Information der Magnetresonanzvorrichtung, und
- Ausführen der zumindest einen Applikation, wobei mittels eines Steuerkanals der Schnittstelle zumindest eine Steuerinformation von der zumindest einen Applikation der Magnetresonanzvorrichtung zu einem Ausführen der zumindest einen Applikation bereitgestellt wird.

2. Verfahren nach Anspruch 1,
**dadurch gekennzeichnet, dass** die Schnittstelle dazu ausgebildet ist, die für die zumindest eine Applikation erforderlichen Informationen und/oder Daten bereitzustellen und die Informationen und/oder Daten, die zur Ausführung der zumindest einen Applikation erforderlich sind, der Magnetresonanzvorrichtung bereitzustellen.

3. Verfahren nach einem der vorhergehenden Ansprüche,
**dadurch gekennzeichnet, dass** die zumindest eine Hardware-Information der Magnetresonanzvorrichtung eine Magnetinformation eines Grundmagneten und/oder eine Gradienteninformation eines Gradienten-Systems und/oder eine Sendeinformation eines Hochfrequenzsystems und/oder Empfangsinformation des Hochfrequenzsystems und/oder eine Information einer Rekonstruktionseinheit.

4. Verfahren nach einem der vorhergehenden Ansprüche,
**dadurch gekennzeichnet, dass** mittels zumindest eines Informationskanals der Schnittstelle eine von der zumindest einen Applikation bereitgestellte Information zur Darstellung an eine Benutzerschnittstelle der Magnetresonanzvorrichtung bereitgestellt wird.

5. Verfahren nach Anspruch 4,
**dadurch gekennzeichnet, dass** mittels des zumindest einen Informationskanals der Schnittstelle eine Information einer Benutzerinteraktion bezüglich der von der zumindest einen Applikation bereitgestellten Information der zumindest einen Applikation bereitgestellt wird.

6. Verfahren nach einem der Ansprüche 4 bis 5,
**dadurch gekennzeichnet, dass** die von der zumindest einen Applikation bereitgestellte Information eine grafische Oberfläche einer Anzeige umfasst, wobei die grafische Oberfläche mittels der Benutzerschnittstelle der Magnetresonanzvorrichtung darstellbar ist.

7. Verfahren nach einem der vorhergehenden Ansprüche,
**dadurch gekennzeichnet, dass** die zumindest eine Applikation Scanner-unabhängig aufgebaut ist.

8. Verfahren nach einem der vorhergehenden Ansprüche,
**dadurch gekennzeichnet, dass** die zumindest einen Applikation für ein Ausführen der zumindest einen Applikation eine Steuerinformation für eine Ansteuerung zumindest einer Hardware-Komponente der Magnetresonanzvorrichtung mittels des Steuerkanals der Schnittstelle bereitstellt.

9. Verfahren nach einem der vorhergehenden Ansprüche,
**dadurch gekennzeichnet, dass** vor einem Ausführen der zumindest einen Applikation und/oder während eines Ausführens der zumindest einen Applikation mittels eines Informationskanals der Schnittstelle dynamische Steuerinformationen der Magnetresonanzvorrichtung der zumindest eine Applikation bereitgestellt werden.

10. Verfahren nach einem der vorhergehenden Ansprüche,
**dadurch gekennzeichnet, dass** mittels eines Informationskanals der Schnittstelle Rohdaten einer Magnetresonanzmessung zu einer Rekonstruktion der zumindest einen Applikation bereitgestellt werden.

11. Verfahren nach einem der vorhergehenden Ansprüche,
**dadurch gekennzeichnet, dass** mittels eines Informationskanals der Schnittstelle rekonstruierte Magnetresonanzbilddaten von der zumindest einen Applikation bereitgestellt werden.

12. Verfahren nach einem der vorhergehenden Ansprüche,
**dadurch gekennzeichnet, dass** die Schnittstelle direkt an einer Systemsteuereinheit der Magnetresonanzvorrichtung angeordnet ist und/oder mittels eines Netzwerks mit der Systemsteuereinheit der Magnetresonanzvorrichtung verbunden ist.

13. Verfahren nach einem der vorhergehenden Ansprüche,
**dadurch gekennzeichnet, dass** die Schnittstelle ein Lizenzmodul aufweist.

14. Verfahren nach einem der vorhergehenden Ansprüche,
**dadurch gekennzeichnet, dass** die Schnittstelle ein Lernmodul umfasst, wobei das Lernmodul ein künstliches neuronales Netzwerk umfasst.

15. Magnetresonanzvorrichtung mit einer Schnittstelle, wobei die Magnetresonanzvorrichtung mittels der Schnittstelle zu einem Ausführen des erfindungsgemäßen Verfahrens nach einem der vorhergehenden Ansprüche ausgebildet ist.

16. System mit einer Magnetresonanzvorrichtung nach Anspruch 15 und zumindest einer Applikation, wobei das System zu einem Ausführen des erfindungsgemäßen Verfahrens nach einem der Ansprüche 1 bis 14 ausgebildet ist.
